Europäisches Patentamt

(19) European Patent Office

Office européen des brevets

(11) Publication number : **0 289 229 B1**

(12) **EUROPEAN PATENT SPECIFICATION**

(45) Date of publication of patent specification :
29.01.92 Bulletin 92/05

(21) Application number : **88303651.9**

(22) Date of filing : **22.04.88**

(51) Int. Cl.⁵ : **A61K 37/52,** A61K 31/70,
// (A61K37/52, 31:70)

(54) **Antiviral combinations.**

(30) Priority : **24.04.87 US 42153**

(43) Date of publication of application :
**02.11.88 Bulletin 88/44**

(45) Publication of the grant of the patent :
**29.01.92 Bulletin 92/05**

(84) Designated Contracting States :
**AT BE CH DE ES FR GB GR IT LI LU NL SE**

(56) References cited :
**EP-A- 0 108 285**
**EP-A- 0 196 185**
**US-A- 4 097 337**
**US-A- 4 178 212**

(73) Proprietor : **THE WELLCOME FOUNDATION**
**LIMITED**
**Unicorn House 160 Euston Road**
**London NW1 2BP (GB)**

(72) Inventor : **Krenitsky, Thomas Anthony**
**106 Laurel Hill Road**
**Chapel Hill North Carolina 27514 (US)**
Inventor : **Zimmerman, Thomas Paul**
**1415 Brunson Court**
**Cary North Carolina 27511 (US)**
Inventor : **Furman, Phillip Allen**
**901 Bluestone Road**
**Durham North Carolina 27713 (US)**

(74) Representative : **Garrett, Michael et al**
**The Wellcome Research Laboratories Group**
**Patents and Agreements Langley Court**
**Beckenham Kent BR3 3BS (GB)**

## Description

The present invention relates to new antiviral combinations for the treatment of prophylaxis of virus infections, especially viruses of the retrovirus or herpes groups.

During the last ten years or more, various antiviral chemotherapeutic agents have been developed for clinical evaluation. A problem with the development of such agents is that, unlike bacteria, viruses are not free living organisms and are dependent for replication on the life processes of the host cell which they are infecting. It is therefore highly desirable for the antiviral agent to exert its effect specifically on the replicative processes of the virus rather than on the corresponding processes of normal (non-infected) cells. The antiviral agents so far developed act via a variety of mechanisms to exert their antiviral effect, such mechanisms involving inhibition of different stages in the process of viral replication in the host cells.

One particular stage of replication at which the virus is susceptable to inhibition is the stage of nucleic acid replication. Thus, for example, in the case of retroviruses, such as Human Immunodeficiency Virus (HIV), the causative virus of Acquired Immune Deficiency Syndrome (AIDS), the production of new retroviral DNA involves the interactions of the enzyme reverse transcriptase (RNA dependent-DNA polymerase) with the constituent nucleotides (specifically desoxyribonucleotides) which act as building blocks for the new retroviral DNA. In the case of DNA viruses, such as herpes viruses, the production of the new viral DNA involves the interaction of the enzyme DNA polymers with the constituent nucleotides (specifically desoxyribonucleotides) which act as building blocks for the new viral DNA. Antiviral action at this stage generally involves the metabolism of nucleotide analogues to "fraudulent" or deleterious nucleotides which mimic the normal physiological substrates and either compete for retroviral DNA polymerase (reverse transcriptase) or, in the case of DNA viruses, DNA polymerase or are incorporated into the viral DNA chain to render it non-functional.

These "fraudulent" or deleterious nucleotides often comprise a nucleoside triphosphate derived from a nucleoside analogue which is converted by enzymes, firstly into the monophosphate and then subsequently into the diphosphate and finally into the triphosphate form. The conversion of the nucleoside analogue to its monophosphate is effected by a thymidine kinase which may be a normal cellular enzyme (i.e. present in uninfected cells) or a virally specified enzyme, depending upon the nucleoside analogue and the infecting virus. One example of this type of antiviral agent is zidovudine (3′-azido-3′-deoxythymidine) which is related to the naturally occurring nucleoside, thymidine, but which contains an azido groups ($N_3$), rather than hydroxy (OH) in the 3′-position of the sugar moiety. Zidovudine is converted sequentially to its mono-, di- and triphosphate forms by normal cellular enzymes. The 5′-triphosphate of zidovudine serves as an inhibitor of reverse transcriptase since it resembles the natural nucleotide substrate, deoxythymidine 5′-triphosphate (dTTp), and as a result competes with dTTP for binding to the reverse transcriptase. It thus competitively inhibits the effectiveness of the enzyme and consequently inhibits viral replication. When the 5′-triphosphate of zidovudine acts as a substrate for reverse transcriptase, it becomes incorporated into the viral DNA chain but, since it contains an azido group rather than the normal hydroxyl group at the 3′-position of the sugar moiety, it is believed to act as a DNA chain terminator whereby viral replication is prevented.

Thus, the antiviral effect of zidovudine, and related compounds which operate via an analogous mode of action, involves competitive inhibition of the virus-coded reverse transcriptase and/or premature chain termination of the viral DNA chain.

Another example of this type of antiviral is acyclovir, (i.e. 9-[(2-hydroxyethoxy)methyl]guanine), which is related to the naturally occurring nucleoside, guanosine, but which contains an acyclic side-chain in the 9-position instead of a cyclic sugar residue in this position, as in guanosine. The antiviral mechanisms of action of acyclovir involves first its permeation of the cell membrane and then its conversion to acyclovir monophosphate by the virally specified enzyme thymidine kinase. Once formed, acyclovir monophosphate is converted by normal cellular enzymes (kinases) to the diphosphate and subsequently to acyclovir triphosphate (ACVTP). Acyclovir triphosphate serves as an inhibitor of viral DNA polymerase since it resembles the natural nucleotide substrate, deoxyguanosine triphosphate (dGTP), and as a result competes with dGTP for binding to the DNA polymerase and thus competively inhibits the effectiveness of the enzyme and consequently viral replication. When ACVTP acts as a substrate for DNA polymerase it becomes incorporated into the viral DNA chain but since it lacks the 3′-hydroxy group of the cyclic sugar moiety it acts as a DNA chain terminator. It also apparently inactivates the viral DNA polymerase. Viral replication is thereby prevented.

Thus, the antiviral effect of acyclovir, and related compounds which operate via an analogous mode of action, involves competitive inhibition, premature chain termination and apparent inactivation of the viral DNA polymerase.

A disadvantageous aspect of a nucleoside analogue is that the naturally occurring substrate for the relevant enzyme may compete at one or more of the enzymatic steps involved in the metabolism of the analogue to its biologically active form or may antagonize the effect of the active metabolite. In this manner, the build up of,

2

for example, dTTP may hinder the binding of zidovudine 5′-triphosphate to the polymerase and thereby prevent the subsequent termination of viral DNA processing. As another example, thymidine may compete with zidovudine for phosphorylation of thymidine kinase and may, therefore, reduce the cellular formation of one of the intermediate metabolites (zidovudine 5′-monophosphate) required for the final active metabolite (zidovudine 5-triphosphate) and this nucleoside analogue.

In similar manner, the buildup of, for example, thymidine may hinder the binding of acyclovir to the virally specified thymidine kinase and thereby antagonise the subsequent phosphorylation of acyclovir, which phosphorylation has been shown to be an essential step for the antiviral action of this drug.

We have now discovered that the use of thymidine phosphorylase in conjunction with an antiviral agent of the above described type i.e. a nucleoside which is phosphorylated to the monophosphate by a thymidine kinase, gives a surprisingly large potentiation of the antiviral activity of the antiviral agent. We believed this is due to the restriction by the thymidine of the influx of physiologically occurring thymidine, and the consequent increase in the ratio of the antiviral compound to the competing nucleoside substrate of thymidine kinase thereby enhancing the phosphorylation of the antiviral compound. The net result is that the use of thymidine phosphorylase in combination with an antiviral agent of the above described type results in a surprising synergistic increase in antiviral efficacy in comparison with the individual antiviral effects of the components of the combination. Indeed, thymidine phosphorylase may exhibit no antiviral effect whatsoever.

The present invention has been found to be particularly applicable to the treatment of asymptomatic infections or disease caused by or associated with human retroviruses, as described below.

According to the present invention we provide a combination of (a) an antiviral compound selected from (i) 9-[(2-hydroxyethoxy)methyl]guanine and physiologically acceptable salts and esters thereof, and (ii) zidovudine and physiologically acceptable salts and esters thereof; and (b) a thymidine phosphorylase; components (a) and (b) of the combination being employed in a ratio whereby a synergistic antiviral effect is achieved.

The term "synergistic antiviral effect" is used to denote an antiviral effect which is greater than the predicted purely additive effects of the individual above-defined components (a) and (b) of the combination.

According to a second feature of the invention there is provided a combination as described above for use in therapy, particularly for the treatment or prophylaxis of viral infections.

The above-described combinations may advantageously be employed for example in the treatment or prophylaxis of herpes virus infections, especially herpes simplex, varicella zoster, cytomegalo-virus (CMV) and Epstein-Barr virus (EBV) infections.

Examples of retroviral infections which may be treated or prevented in accordance with the invention include human retroviral infections such as Human Immunodeficiency Virus (HIV), HIV-2 and Human T-Cell Lymphotropic Virus (HTLV) e.g. HTLV-I or HTLV-IV infections. The compounds according to the invention are especially useful for the treatment or prophylaxis of AIDS and relates clinical conditions such as AIDS-related complex (ARC), progressive generalised lymphadenopathy (PGL), AIDS-related neurological conditions, such as multiple sclerosis or tropical paraparesis, anti-HIV antibody-positive and HIV-positive conditions, Kaposi's sarcoma and thrombocytopenia purpura. The compounds may also be used in the treatment or prevention of psoriasis.

The above-described combinations may also advantageously be employed in the treatment of prophylaxis of retroviral infections.

It will be appreciated that in accordance with the present invention the antiviral compound and the thymidine phosphorylase may be administered substantially simultaneously for example in separate formulations, or sequentially, preferably by different routes. In the latter case, however, the components of the combination are administered within a sufficiently short interval to ensure that a synergistic antiviral effect is achieved.

An advantage of the combination according to the invention is that it enables one to obtain an improved antiviral efficacy at a particular dosage of the antiviral compound (compared with the compound used alone) thereby improving the therapeutic index of the compound. Thus, for example, the combination may be used to treat conditions which would otherwise require relatively large dosages of the antiviral compound at which the toxicity problems may occur. The smaller dosages of the combination may provide for increased convenience to the patient and increased compliance.

With regard to the antiviral compound, this is generally selected from compounds that are enzymatically phosphorylated by a thymidine kinase in vivo to give a nucelotide which is an inhibitor of the viral DNA-polymerase and/or is incorporated into the viral DNA chain to make it non-functional. Such compounds are generally substrates for an appropriate kinase enzyme which phosphorylates the compounds to form initially a monophosphate which is then phosphorylated (also by kinase enzymes) first to the diphosphate and finally to the triphosphate which interacts deleteriously with the DNA-polymerase.

a-[(2-Hydroxyethoxy)methyl]guanine and its physiologically acceptable salts and esters employed in the above combinations can be obtained by processes that are described in the literature for example, U.K. Patent Specification 1523865.

Examples of the above-mentioned esters include acyl esters such as straight or branched chain acyl (e.g., $C_{2-18}$), aracyl (e.g., phenylacetyl), aroyl (e.g., benzoyl optionally substituted by halogen, $C_{1-4}$ alkyl or $C_{1-4}$ alkoxy), organosulphonyl (e.g., methanesulphonyl) and mono-, di- or triphosphate esters as well as the corresponding thio esters including dithiocarbamyl, e.g., dialkyl (e.g., methyl) dithiocarbamoyl, esters.

Examples of the above-mentioned salts include base salts, e.g., derived from an appropriates base, such as alkali metal (e.g. sodium) or alkaline earth metal salts.

Zidovudine and its physiologically acceptable salts and esters may be prepared in conventional manner for example as described in the following references or by methods analogous thereto, J.R. Horwitz et al., J. Org. Chem., 29, (July 1984) 2076-78, and M. Imazawa et al., J. Org. Chem., 43, (15) (1978) 3044-3048; K.A. Watanabe et al., J. Org. Chem., 45, 3274, (1980); or R.P. Glinski et al., J. Chem. Soc., Chem. Commun., 915, (1970) and also European Patent Specification No. 196185.

The thymidine phosphorylase for use in accordance with the present invention is preferably of bacterial origin. The production and purification of such thymidine phosphorylase is described in U.S. patents US-A-4,097,337 and US-A-4,178,212.

The present invention further includes a process for preparing the above-defined combinations according to the invention which comprises bringing into association an above-defined antiviral compound and thymidine phosphorylase to provide a synergistic antiviral effect.

The combinations according to the invention may be administered to the subject concerned in conventional manner. As indicated above, the antiviral compound and the thymidine phosphorylase may be administered simultaneously (e.g., in a unitary pharmaceutical formulation) or separately (e.g., in separate pharmaceutical formulations). In general, the respective components of the combinations may be administered by the topical, oral, rectal or parenteral (e.g. intravenous, subcutaneous or intramuscular) route. The dosage of the combination will depend on the condition being treated, the particular antiviral agent and other clinical factors such as the weight and condition of the patient and the route of administration of the compound. However, for administration by the oral route a dosage of the antiviral compound of 1 to 200 mg/kg/day, preferably 5 to 50 mg/kg/day, is generally sufficient. For administration by the parenteral route, a dosage of antiviral compound of 1 to 100 mg/kg/day, preferably 2 to 30 mg/kg/day is generally sufficient. The amount of thymidine phosphorylase in the combination is preferably in the range of about 0.1 to 100 units of enzyme activity per kilogram of body weight per day and particularly in the range of about 1 to 20 units of enzyme activity per kilogram of body weight per day.

The ratios of the antiviral compound to the thymidine phosphorylase in terms of mg: units of enzyme activity for use according to this invention is from 1:100 to 100:0.1, preferably from 1:20 to 60:1.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by conventional pharmaceutical techniques. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers of both, and then, if necessary, shaping the product.

Formulations of the present invention suitable for oral administration may be presented as discrete units such as capsules, cachets or tablets each containing predetermined amounts of the active ingredients; as powders or granules; as solutions or suspension in an aqueous liquid or a non-aqueous liquid; or as oil-in-water emulsions or a water-in-oil emulsions. A tablet may be made by compression or moulding, optionally with one or more accessory ingredients. Compressed tablets may be prepared by compressing in a suitable machine the active ingredients in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues. e.g, mouth and skin, the formulations are preferably applied as a topical ointment or cream containing the antiviral active ingredient in an amount of, for example. 0.075 to 20% w/w, preferably 0.2 to 15% w/w and most preferably 0.5 to 10% w/w. When formulated in an ointment, the active ingredients may be employed with either paraffinic or a water-miscible ointment base. The active ingredients also may be formulated in a cream with an oil-in-water cream base. Alternatively, the thymidine phosphorylase may be administered topically while the antiviral agent is administered separately by another route (e.g., orally, rectally, intraveneously, subcutaneously or intramuscularly).

If desired, the aqueous phase of the cream base may include, for example, at least 30% w/w of the polyhydric alcohol, i.e. an alcohol having two or more hydroxyl groups such as propylene glycol, butan-1,3-diol, mannitol, sorbitol, glycerol and polyethylene glycol and mixtures thereof. The topical formulation may desirably include a compound which enhances absorption or penetration of the active ingredient through the skin or other affected areas. Examples of such dermal penetration enhancers include dimethylsulphoxide.

The oil phase of the emulsions of this invention may be constituted form known ingredients in a known-manner. While the phase may comprise merely an emulsifier, it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up to so-called emulsifying wax, and the wax together with the oil and/or fat make up the so-called emulsifying ointment base which forms the oily dispersed phase of the creams formulations.

Emulsifiers and emulsion stabilizers suitable for use in the formulations of the present invention include Tween 60®, Span 80®, cetostearyl alcohol, myristyl alcohol, glycerol mono-stearate and sodium lauryl sulphate.

Formulations suitable for parenteral administration include aqueous and non-aqueous sterile injection solutions which may contain anti-oxidants, buffersm bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of the active ingredients.

It should be understood that in addition to the ingredients particularly mentioned above the formulations of this invention may include other agents conventional in the art having regard to the type of formulation in question, for example, those suitable for oral administration may include flavouring agents.

The following Examples illustrate the present invention.

Antiviral Activity

a) Anti-Retroviral Activity

The enhancement of the Anti-Friend Leukemia Virus (FLV) activity of zidovudine by bacterial thymidine phosphorylase is shown in Table 1. FG-10 cells were used seeded on a plate one day before they were infected with FLV. One hour after infecting, known concentrations of the test compound or combination were added. The plates were incubated for 3 days, the media replaced with fresh McCoy's 5A media and incubated another 3 days. Concentrations of test compound/combinations giving 50% inhibition of plaques ($IC_{50}$ nM) were determined as shown in Table 1.

## Table 1

| Compound/Combination | $IC_{50}$ (nM) |
|---|---|
| Zidovudine | 5 |
| Zidovudine + thymidine phosphorylase (40 units of enzyme activity per ml) | >0.001 |

b) Anti-Herpes Activity

Antiviral activity was determined using a plaque reduction assay. Petri plates were seeded with Vero cells which were then allowed to grow to confluency. Each plate was then infected with a fixed number of plaque forming units (abut 100-500) of herpes simplex type 1 (KOS strain). The inhibitors, either alone or in the indicated combinations, were dissolved to give the indicated concentration in minimal essential medium containing 2% heat inactivated fetal calf serum and 0.5% human immune serum globulin: One hour after infection the solutions (10 ml per plate) were added to the cultures. Three days later the cultures were formalin-fixed and stained with crystal violet, and the plaques were counted.

## Table 2

| Compound/Combination | Conc. (μM) | Average Plaque Count | % Inhibition |
|---|---|---|---|
| Acyclovir | 0.1 | 316.5 | 11.6 |
| | 0.25 | 298 | 16.7 |
| | 0.5 | 290.5 | 19.0 |
| | 1.0 | 230 | 35.8 |
| | 2.5 | 182 | 49.2 |
| | 5.0 | 88 | 75.4 |
| | 7.5 | 54 | 84.9 |
| | 10 | 34.5 | 90.4 |
| Acyclovir + thymidine phosphorylase (40 units/ml) | 0.1 | 232 | 35.2 |
| | 0.25 | 162.5 | 54.6 |
| | 0.5 | 81 | 77.4 |
| | 1.0 | 3 | 99.2 |
| | 2.5 | 1 | 99.7 |
| | 5.0 | 0 | 100 |
| | 7.5 | 0 | 100 |
| | 10 | 0 | 100 |

No activity against herpes simplex type 1 (KOS strain) for thymidine phosphorylase was noted at concentrations up to 400 units/ml.

Pharmaceutical Formulation

In the following Example, the antiviral compound is zidovudine or acyclovir.

Example

| Cream | Weight |
|---|---|
| Thymidine phosphorylase | 100 units |
| Antiviral compound | 5.00 g |
| Glycerol | 2.00 g |
| Cetostearyl alcohol | 6.75 g |
| Sodium lauryl sulphate | 0.75 g |
| White soft paraffin | 12.50 g |
| Liquid paraffin | 5.00 g |
| Chlorocresol | 0.10 g |
| Purified Water    to | 100.00 g |

The active compounds are dissolved in a mixture of purified water and glycerol and heated to 70°C. The remaining ingredients are heated together at 70°C. The two parts are added together and emulsified. The mixture is cooled and filled into containers.

**Claims**

**Claims for the following Contracting States: AT, BE, CH, DE, FR, GB, IT, LI, LU, NL, SE**

1. A combination of
(a) an antiviral compound selected from (i) 9-[(2-hydroxyethoxy) methyl]guanine and physiologically acceptable salts and esters thereof, and (ii) zidovudine and physiologically acceptable salts and esters thereof; and
(b) a thymidine phosphorylase;
components (a) and (b) of the combination being employed in a ratio whereby a synergistic antiviral effect is achieved.

2. A combination as claimed in claim 1 wherein the thymidine phosphorylase is of bacterial origin.

3. A combination as claimed in claim 1 or claim 2 wherein the ratio of antiviral compound to thymidine phosphorylase (in terms of mg: units of enzyme activity) is in the range of 1:100 to 100:0.1.

4. A combination as claimed in claim 3 wherein the said ratio is in the range of 1:20 to 60:1.

5. A combination as claimed in any of the preceding claims for use in the medical therapy.

6. A combination as claimed in claim 5 for use in the treatment or prophylaxis of viral infections.

**Claims for the following Contracting States: ES, GR**

1. A process for the preparation of combination of
(a) an antiviral compound selected from (i) 9-[(2-hydroxyethoxy) methyl]guanine and physiologically acceptable salts and esters thereof, and (ii) zidovudine and physiologically acceptable salts and esters thereof; and
(b) a thymidine phosphorylase;
which comprises bringing components (a) and (b) of the combination into association in a ratio whereby a synergistic antiviral effect is achieved.

2. A process as claimed in claims 1 wherein the thymidine phosphorylase is of bacterial origin.

3. A process as claimed in any of the preceding claims wherein the ratio of antiviral compound to thymidine phosphorylase (in terms of mg: units of enzyme activity) is in the range of 1:100 to 100:0.1.

4. A process as claimed in claim 3 wherein the said ratio is in the range of 1:20 to 60:1.

**Patentansprüche**

**Patentansprüche für folgende Vertragsstaaten: AT, BE, CH, DE, FR, GB, IT, LI, LU, NL, SE**

1. Kombination von
(a) einer antiviralen Verbindung, ausgewählt aus (i) 9-[(2-Hydroxyethoxy)-methyl]-guanin und physiologisch annehmbaren Salzen und Ester hievon, und (ii) Zidovudin und physiologisch annehmbaren Salzen und Ester hievon; und
(b) einer Thymidinphosphorylase;
wobei die Komponenten (a) und (b) der Kombination in einem einen synergistischen antiviralen Effekt erzielenden Verhältnis eingesetzt werden.

2. Kombination nach Anspruch 1, wobei die Thymidinphosphorylase bakteriellen Ursprungs ist.

3. Kombination nach Anspruch 1 oder 2, wobei das Verhältnis von antiviraler Verbindung zu Thymidinphosphorylase (in Form von mg : Enzymaktivitätseinheiten) im Bereich von 1:100 bis 100:0,1 beträgt.

4. Kombination nach Anspruch 3, wobei das Verhältnis im Bereich von 1:20 bis 60:1 beträgt.

5. Kombination nach einem der vorhergehenden Ansprüche zur Verwendung in der medizinischen Therapie.

6. Kombination nach Anspruch 5 zur Verwendung in der Behandlung oder Prophylaxe von Virusinfektionen.

**Patentansprüche für folgende Vertragsstaaten: ES, GR**

1. Verfahren zur Herstellung einer Kombination von
(a) einer antiviralen Verbindung, ausgewählt aus (i) 9-[(2-Hydroxyethoxy)-methyl]-guanin und physiologisch annehmbaren Salzen und Ester hievon, und (ii) Zidovudin und physiologisch annehmbaren Salzen und Ester hievon; und
(b) einer Thymidinphosphorylase;
umfassend das in Berührung bringen der Komponenten (a) und (b) der Kombination in einem einen synergistischen antiviralen Effekt erzielenden Verhältnis.

2. Verfahren nach Anspruch 1, wobei die Thymidinphosphorylase bakteriellen Ursprungs ist.

3. Verfahren nach einem der vorhergehenden Ansprüche, wobei das Verhältnis von antiviraler Verbindung zu Thymidinphosphorylase (in Form von mg : Enzymaktivitätseinheiten) im Bereich von 1:100 bis 100:0,1 beträgt.

4. Verfahren nach Anspruch 3, wobei das Verhältnis im Bereich von 1:20 bis 60:1 beträgt.

**Revendications**

**Revendications pour les Etats contractants suivants: AT, BE, CH, DE, FR, GB, IT, LI, LU, NL, SE**

1. Mélange :
(a) d'un composé antiviral choisi entre (i) la 9-[(2-hydroxyéthoxy)méthyl]guanine et ses sels et esters physiologiquement acceptables, et (ii) la zidovudine et ses sels et esters physiologiquement acceptables, et
(b) d'une thymidine phosphorylase;
les composants (a) et (b) du mélange étant utilisés dans un rapport auquel un effet antiviral synergique est atteint.

2. Mélange suivant la revendication 1, dans lequel la thymidine phosphorylase est d'origine bactérienne.

3. Mélange suivant la revendication 1 ou 2, dans lequel le rapport du composé antiviral à la thymidine phosphorylase (en mg:unités d'activité enzymatique) se situe dans l'intervalle de 1:100 à 100:0,1.

4. Mélange suivant la revendication 3, dans lequel le rapport se situe dans l'intervalle de 1:20 à 60:1.

5. Mélange suivant l'une quelconque des revendications précédentes, à utiliser en thérapeutique médicale.

6. Mélange suivant la revendication 5, à utiliser dans le traitement ou la prophylaxie d'infections virales.

**Revendications pour les Etats contractants suivants: ES, GR**

1. Procédé de préparation d'un mélange :
(a) d'un composé antiviral choisi entre (i) la 9-[(2-hydroxyéthoxy)méthyl]guanine et ses sels et esters physiologiquement acceptables, et (ii) la zidovudine et ses sels et esters physiologiquement acceptables, et

(b) dune thymidine phosphorylase;
qui comprend la mise en association des composants (a) et (b) du mélange dans un rapport auquel un effet antiviral synergique est atteint.

2. Procédé suivant la revendication 1, dans lequel la thymidine phosphorylase est d'origine bactérienne.

3. Procédé suivant l'une quelconque des revendications précédentes, dans lequel le rapport du composé antiviral à la thymidine phosphorylase (en mg:unités d'activité enzymatique) se situe dans l'intervalle de 1:100 à 100:0,1.

4. Procédé suivant la revendication 3, dans lequel le rapport se situe dans l'intervalle de 1:20 à 60:1.